(19) Europäisches Patentamt
European Patent Office
Office européen des brevets

(11) Publication number: **0 206 740**
**A1**

(12) # EUROPEAN PATENT APPLICATION

(21) Application number: **86304664.5**

(22) Date of filing: **17.06.86**

(51) Int. Cl.[4]: **C 07 C 179/10**, C 07 C 178/00

(30) Priority: **21.06.85 US 747469**

(43) Date of publication of application: **30.12.86 Bulletin 86/52**

(84) Designated Contracting States: **AT BE CH DE FR GB IT LI NL**

(71) Applicant: **THE PROCTER & GAMBLE COMPANY, One Procter & Gamble Plaza, Cincinnati Ohio 45202 (US)**

(72) Inventor: **Dyer, John Collins, 9 Darby Heath, Fairfield Ohio 45014 (US)**

(74) Representative: **Brooks, Maxim Courtney et al, Procter & Gamble (NTC) Limited Whitley Road Longbenton, Newcastle-upon-Tyne NE12 9TS (GB)**

(54) **Preparation of peroxycarboxylic acids and magnesium peroxycarboxylates.**

(57) The invention relates to an improved process for making peroxycarboxylic acids and their corresponding magnesium peroxycarboxylates.

In the process of the invention, an acyl chloride or anhydride is reacted with a source of hydrogen peroxide in an organic solvent in the presence of a phase transfer catalyst. If magnesium ions are also present during the reaction, the corresponding magnesium peroxycarboxcylates are formed.

EP 0 206 740 A1

# PREPARATION OF PEROXYCARBOXYLIC ACIDS AND MAGNESIUM PEROXYCARBOXYLATES

John Collins Dyer

## Technical Field

This invention relates to an improved process for making peroxycarboxylic acids and the corresponding magnesium peroxycarboxylates.

Heretofore, preparation of magnesium peroxycarboxylates has been by the method of first preparing the corresponding peroxy acid compound group and then neutralizing in the presence of a magnesium compound and in the presence of a solvent selected so that the magnesium salt precipitates therefrom. This method entails handling the peroxyacids which involves a certain risk as many are explosive.

One purpose of the invention is to provide a high yield, one step, safe process for the preparation of magnesium peroxycarboxylates which does not involve isolation of the intermediate peroxy acid.

It will be recognized that where reference is made herein to the magnesium salt of a peroxycarboxylic acid, the salt is formed from the peroxycarboxylic acid group and not the carboxylic acid group. These compounds are disclosed in U.S. Patent 4,483,781, Hartman, issued November 20, 1984, said patent being incorporated herein by reference.

## Summary of the Invention

The invention relates to an improved process for making peroxycarboxylic acids and their corresponding magnesium peroxycarboxylates.

In the process of the invention, a peroxyacid precursor, preferably an acyl chloride or anhydride is reacted with hydrogen peroxide or another source of peroxide in an organic solvent in the presence of a phase transfer catalyst. The reaction mixture is stirred, and the precipitate filtered. Analysis shows a high percentage of peroxycarboxylic acid present in the product solution. If magnesium salts are also present during the reaction, the corresponding magnesium peroxycarboxylate is formed.

## Detailed Description of the Invention

The basic structure of the compounds prepared by the process described herein is that of a peroxycarboxylic acid or magnesium peroxycarboxylate of the following formulas. The peroxycarboxylic acid has the general formula:

$$R - \overset{O}{\overset{\|}{C}} - OOH$$

wherein R is as hereinafter defined.

The magnesium peroxycarboxylates can be represented by the general formula:

$$R \left[ \overset{O}{\overset{\|}{C}} - O - O - Mg - Z \right]_{w}$$

wherein R is hydrogen (H), oxygen (O-), or an organic moiety, Z is a negatively charged organic or inorganic moiety and W is from about 1 to about 3. Such compounds possess excellent storage stability both alone and when mixed with additional solid components. Such additional solid components can even be alkaline. Stability, as used herein, means that in most instances the active oxygen contained in the magnesium peroxycarboxylates is retained during storage to a much greater extent than is the active oxygen contained in the corresponding peroxycarboxylic acids. The active oxygen contained in the solid magnesium peroxycarboxylates is generally readily available. This means that the solid magnesium peroxycarboxylates are readily soluble or dispersible and yield solutions containing peroxyacids. When the solution is aqueous, it cannot be distinguished from an aqueous solution prepared from the corresponding peroxycarboxylic acid, and an equivalent amount of magnesium salts or magnesium hydroxide, when the compared solutions are adjusted to the same pH.

Preferably R is selected from the group consisting of linear or branched alkyl groups containing from about 1 to about 20

carbon atoms (more preferably from about 8 to about 18), an aryl group, an aromatic heterocyclic group, a polyarylene group consisting of from about 2 to about 4 annelated benzenoid rings, and mixtures thereof. Also, R can be substituted with essentially any group or groups including hydroxy, halogen (chloro, bromo, or fluoro), sulfonate, nitro, carboxylate, phenyl, $C_{1-5}$ alkoxy (e.g. ethoxy), $C_{1-5}$ alkyl, hydroxy, sulfonyl, aryl, heteroaryl, sulfone, amine oxide, ammonium and substituted ammonium ($R^1_4N+$, wherein $R^1$ is as hereinafter defined), amide, ester, nitrile, and sulfate groups to replace a hydrogen atom attached to the alkyl or aryl portions of the R moiety. R may not contain substituents which would react readily with the active oxygen from the percarboxylate groups. Such reactive groups can include electrophilic groups such as ketones, sulfoxides, reactive esters of alpha, beta-unsaturated carbonyls, nitriles, etc. Preferably, the peroxycarboxylic acid has a melting point above about 50°C., most preferably above about 60°C.

The R group may be covalently bonded to other R groups to form a polymer. Typically, R in this case is an alkylene, e.g., vinyl, group substituted with the magnesium peroxycarboxylate moiety. Usually the number of repeating R groups, m, averages from about 2 to about 100.

It is preferred that Z have the general formula:

$$R - \overset{O}{\overset{\|}{C}} - O - O-$$

wherein R is as defined above. Such a selection of Z is preferred because twice as much active oxygen per mole of magnesium is present than in examples where Z contains no active oxygen. R and Z can be covalently bonded, as in the case of a diperoxycarboxylic acid. However, Z can also be a corresponding carboxylate, sulfate, sulfonate, chloride, bromide, nitrate, etc. anion. The anion obviously should be one that is not readily oxidized by the peroxycarboxylate.

The more preferred R groups consist of branched and linear alkyl groups containing at least 8 carbon atoms preferably from

about 8 to about 14 carbon atoms or a substituted aryl group of the types shown below:

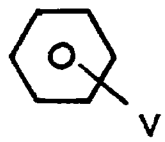

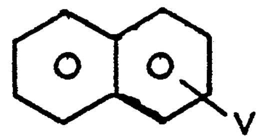

wherein each V is a substituent selected from the group including $-NH_3^+$, $-NR_3^+$, F, Cl, Br, $-SO_3^-M^+$, $-CO_2^-M^+$, $-NO_2$, $-OCH_3$, $-OC_4H_9$, $-CH_3$, $-CF_3$, $-C\equiv N$ and H where M is an alkali or alkaline earth metal.

The most preferred R groups are as follows:

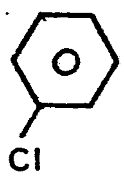 ; 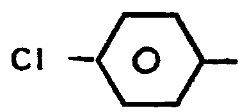 ; 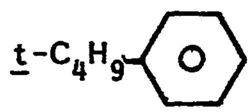 ;

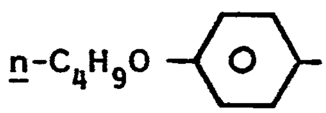 $n-C_{11}H_{23}-$; $-(CH_2)_{10}-$; and

and $n-C_{10}H_{21}-CH-$
$CH_2$

The more preferred Z groups are:

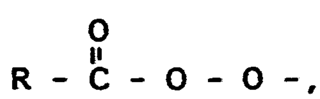, 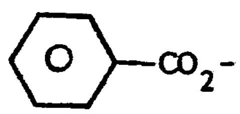, $CH_3CO_2-$,

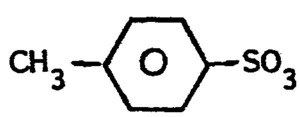, 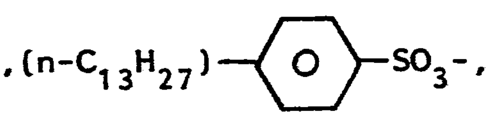,

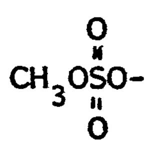, Cl- and 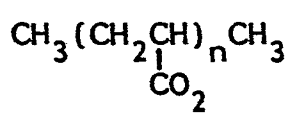

where n is approximately 50.

The most preferred Z groups are Cl- and

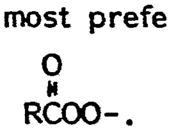.

The process of the invention involves the extraction of hydrogen peroxide or its anion(s) into an organic phase containing a peroxy acid precursor; such as an acid chloride or anhydride described hereinafter. The extraction is effected by the use of a phase transfer catalyst described hereinafter. Following the extraction, the hydrogen peroxide anion reacts with the peroxy acid precursor to generate the desired peroxycarboxylic acid in the organic phase where it is easily separable from the reaction mixture by crystallization, evaporation, or filtration.

The precipitate formed from the reaction described hereinbefore is initially a wet solid. It is believed to be desirable under some circumstances, e.g., when the peroxycarboxylic acid is relatively insoluble, to add to this wet amorphous solid other agents which can help disperse the product once dried or to help to maintain the state of hydration, or for any other reason. The addition of these other agents can be conveniently effected at this stage of the process before the solid magnesium peroxycarboxylate is dried as hereinbefore described. Examples of such additives include polymers of acrylic acid and its neutralized salts having a molecular weight maximum of between 500 and 80,000. These polymers of acrylic acid or its neutralized salts can be added as an aqueous solution or suspension or as a solid which is intermixed with the wet product magnesium peroxycarboxylate. Other additives can be selected from the group consisting of 4-toluenesulfonic acid or its sodium salt, polyvinylpyrrolidone, polystyrene sulfonate, polyethylene glycols, sodium sulfate, magnesium salts of weak acids having pKa values of between about 3.5 and about 5.5 and detergent surfactant molecules selected from the group which includes linear alkylbenzene sulfonates, alcohol sulfates, linear alkyl ethoxylates, alkyl glycosides, and mixtures thereof. The preferred alkyl chain length in those detergent surfactants is from about 10 to about 18 carbon atoms.

The products are obtained after the drying steps described hereinbefore are typically in the form of fine powders. It is

often desirable to increase the particle size of these products so as to control dustiness or prevent or reduce the amount of segregation that occurs when the product is admixed with larger compounds having other particle sizes. The products of the process described hereinbefore which are magnesium peroxycarboxylates can be agglomerated or extruded to increase their particle size to a desirable level. Such processes are typically found not to impair the desirable stability characteristics of these products. In addition, increased particle sizes are believed to increase the volume to surface area ratio. This can reduce the relative amount of the magnesium peroxycarboxylate which can be exposed to any external hostile environment. Increased particle sizes are found in many cases to actually increase the stability of the magnesium peroxycarboxylates, particularly when said magnesium peroxycarboxylates are admixed with other compounds which are hostile to the stability of the corresponding peroxycarboxylic acid.

<u>The Peroxy Acid Precursor</u>

The peroxy acid precursor has the general formula:

$$R - \overset{\overset{O}{\|}}{C} - L$$

wherein L is a suitable leaving group replaceable by -OOH and hereinafter defined, and R is as defined hereinbefore. The leaving group L must be sufficiently lobile so that the reaction proceeds expeditiously. The preferred leaving groups include those whose conjugate acids have $pK_a$ values between 0.5 and 10, preferably between 1 and 5. Examples include acid chlorides, anhydrides, or esters wherein L = $Cl^-$, $F^-$, $RCO_2^-$, $RSO_3^-$, $RCO_3^-$, and $C_6H_5O^-$ and R is as defined hereinbefore. The most preferred example is L = $Cl^-$ for reasons of best reactivity and availability. The most preferred R is required to be the same as defined hereinbefore.

<u>The Catalyst</u>

Suitable phase transfer catalysts are well known in the art (e.g. Herriot and Picker, <u>J. Am. Chem. Soc.</u> <u>1975</u>, <u>97</u>, 2345).

Typically the phase transfer catalyst is selected from the group consisting of a polyether and an onium salt including a cationic quaternary of a Group VA element of the Periodic Table, and salts thereof. More preferred are the quaternary ammonium or phosphonium salts thereof. The reaction may be carried out at any temperature within a wide range from about the freezing point of the reaction mass to about the reflux temperature of the solvent, provided it is lower than a temperature which is deleterious to the peroxycarboxylic acid formed. The reaction is of particular interest because it generally proceeds at room temperature or below, at satisfactory speed, and with excellent yields. The reaction may also be carried out at any desired pressure from subatmospheric to superatmospheric, but atmospheric pressure is preferably employed for convenience, and because there appears to be no substantial advantage to be gained from operating at higher pressures.

By "onium salts" I more particularly refer to quaternary ammonium and phosphonium salts such as are generally used in the phase transfer catalysis of heterogeneous reactions in immiscible liquids. The general requirement for the onium salt chosen is that it be soluble in both the organic and aqueous phases, when these two liquid phases are present, and usually a little more soluble in the organic phase than the aqueous phase. The reaction will also proceed with a phase transfer catalyst when there is only a single organic liquid phase present, but such a reaction is less preferable than one in which both aqueous and organic liquid phases are present. A wide variety of onium salts are effective in this synthesis.

The onium salts include the well-known quaternary salts of Group VA elements of the Periodic Table, such as are disclosed in U.S. Pat. No. 3,992,432, in Angewandte Chemie, International Edition in English, 16, 493-558 (August 1977) and in Synthesis 441-456 (1973), all of which are incorporated herein by reference. Discussed therein are various anion transfer reactions where the phase transfer catalyst exchanges its original ion for other ions

in the aqueous phase, making it possible to carry out chemistry there with the transported anion, including $^{-}OOH$ ions.

The onium salts used in this synthesis include one or more groups having the formula $(R_nY)^+X^-$, wherein Y is a tetra coordinate ion derived from an element of Group VA; R is an organic moiety of the salt molecule bonded to Y when Y is tetracoordinate, $X^-$ is a nonoxidizable anion which will dissociate from the cation $(R_nY)^+$ in an aqueous environment. The group $(R_nY)^+X^-$ may be repeated as in the case of dibasic quaternary salts having two tetra coordinate Group VA ions substituted in the manner described, or as in the case wherein $R_n$ is a polymeric chain.

The preferred onium salts for use in the invention have the formula

$$(R^1R^2R^3R^4M^+)X^-$$

wherein M is N or P, and $R_1$-$R_4$ are monovalent hydrocarbon radicals preferably selected from the group consisting of alkyl, aryl, alkaryl, aralkyl, and cycloalkyl moieties or radicals, optionally substituted with suitable heteroatom-containing functional groups. The onium salts are generally selected to be preferentially less soluble in the less polar of the two distinct liquid phases. Any of the salts disclosed in U.S. Pat. No. 3,992,432 will be found effective, but most preferred are those in which the total number of carbon atoms in $R^1$, $R^2$, $R^3$, and $R^4$ cumulatively range from about 12 to about 57, and preferably range from about 13 to about 30. Most preferred onium salts have M=N, and hydrocarbon radicals where $R^1$, $R^2$, $R^3$, and $R^4$ are each selected from the group consisting of n-$C_4H_9$, n-$C_5H_{11}$, mixed $C_5H_{11}$, n-$C_6H_{13}$, mixed $C_6H_{13}$, $C_6H_5$, $C_6H_5CH_2$, n-$C_8H_{17}$, mixed $C_8$-$C_{10}$ alkyl, and the like. Especially preferred is tetra-n-butylammonium.

Various counterions may be used, including $Cl^-$, $HO^-$, $NO_3^-$, $SO_4^=$, $HSO_4^-$ and $CH_3CO_2^-$. Most preferred is $HO^-$.

The polyethers useful as catalysts in this synthesis include cyclic polyethers such as the crown ethers, disclosed in

Agewandte Chemie, supra, and acyclic polyethers having the formula

$$R - O - R'$$

wherein R and R' are independently alkyl having from 1 to about 16 carbon atoms, or alkyl containing substituted functional groups such as hydroxy, ether, etc. Most preferred acyclic polyethers have the formula

$$R-(OCH_2CH_2)_rOR''$$

wherein R is an alkyl group containing from about 1 to about 16 carbon atoms and R" is an alkyl group containing from about 1 to about 16 carbon atoms, or H, and r is an integer in the range from 0 to about 300. Most preferred are commonly available polyethers such as: tetraethylene glycol dimethyl ether; polyethylene oxide (mol wt about 5000); poly(ethylene glycol methyl ether); 1,2-dimethoxyethane; diethyl ether, and the like. A covalent attachment through an oxygen atom between R and R' or R and R" is also preferred. This describes the class of compounds known as "crown ethers". These are described more completely in J. Am. Chem. Soc. 1980, 102, 4810-4815.

The amount of phase transfer catalyst used is critical in controlling the rate of the reaction. Where the rate of the reaction is so high as to develop more heat than can be managed, less catalyst is employed. In general, it is sufficient to use no more onium salt catalyst than about 2-50 percent by weight of the reaction mass, and it is preferred to use in the range from about 1 to about 20 percent by weight. The catalyst can be reused for subsequent reactions.

The Solvent

It is preferred to conduct the reaction in a nonreactive organic solvent. Preferred solvents include ethyl acetate, dichloromethane, sulfolane, tetrahydrofuran, diethyl ether, benzene, hexane, and carbon tetrachloride.

Without wishing to be bound by theory, it is believed that the reaction can be represented by the following scheme:

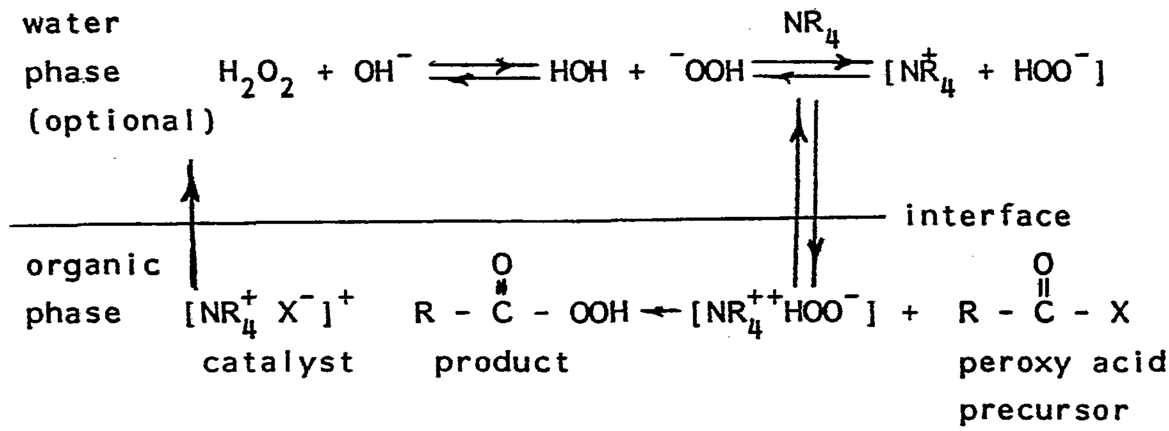


If magnesium peroxide is used rather than hydrogen peroxide the magnesium peroxycarboxylate will be formed. Alternatively, combinations of hydrogen peroxide or sodium peroxide and magnesium salts may be used.

The presence of a discrete water phase is optional within the invention, although a small quantity of moisture is desirable as stated in Dyer, J.C.; Evans, S.A. J. Org. Chem. 1980, 45, 5350-5355.

The products of the process of this reaction, i.e. the peroxycarboxylic acids and magnesium peroxycarboxylates are useful in bleaching compositions and detergent compositions.

The invention is further described in the following nonlimiting illustrative examples.

COMPARATIVE EXAMPLE I

The process described in U.S. Patent 3,321,512 (Cooper and Gibson, May 23, 1967), incorporated herein by reference, in Example I details the conversion of 3-chlorobenzoyl chloride to 3-chloroperoxybenzoic acid in 85% yield and 85% purity. Unlike the processes described within the present invention, the temperature must be controlled by water cooling rather than catalyst level. Also, this process involves more than one reaction vessel and necessitates the isolation of the peroxy acid, whereas the current invention involves only a single reaction vessel and allows the isolation of the more stable hydrated magnesium salt of the peroxy acid.

A similar comparative process described in U.S. Patent 3,235,584, Blumbergs (Feb. 15, 1966), incorporated herein by reference also describes the conversion of organic acid halides to peroxycarboxylic acids.

This process suffers similar constraints to those cited above which are avoided in the present invention.

EXAMPLE I

Ground magnesium peroxide (48% active, 7.4 g, 62 mmol)suspended in ethyl acetate (250 mL) was combined with 4-chlorobenzoyl chloride (10.0 g, 57 mmol) in a round bottom flask. Tetra-n-butyl ammonium hydroxide (40% aqueous, 13.3g, 20 mmol) was added to the stirring suspension. After 60 hours, water (5g) was added. After 1 hour, the product was filtered, washed with ethyl acetate, and air dried to give 7.45 g colorless powder. Titration of an ice cold solution with potassium iodide against sodium thiosulfate showed 7.1% active oxygen as peroxyacid (98% purity as magnesium bis(4-chloroperoxybenzoate) tetrahydrate; 58% recovered chemical yield).

EXAMPLE II

The procedure in Example I was followed exactly except that the level of tetra-n-butyl ammonium hydroxide ($bu_4N^+ OH^-$) was varied with the following results:

| $bu_4N^+ OH^-$ (mmol) | Recovered Filtrate (g) | % Active Oxygen as Peroxyacid | % Purity as Magnesium Salt | Chemical Yield (9%) |
|---|---|---|---|---|
| 5 | 18.2 | 3.7 | 51 | 74 |
| 1 | 18.5 | 1.9 | 26 | 38 |
| 0 | 11.1 | 1.8 | 25 | 22 |

EXAMPLE III

A solution of hydrogen peroxide (98% active) and sodium hydroxide was added to a stirring solution of dichloromethane which contained an equivalent amount (50 mole %) of 4-t-butylbenzoyl chloride. Tetra-n-hexylammonium hydroxide (10 mole %) was added. After 24 h, the two phase reaction mixture was neutralized to pH 3 with sulfuric acid and separated. Removal of the dichloromethane via rotary evaporation gave a high yield of 4-t-butylperoxybenzoic acid and minor amounts of di(4-t-butylbenzoyl) peroxide and residual phase transfer catalyst. The aqueous phase could be recycled into further

reaction batches. The peroxyacid product may be used directly or converted to the hydrated magnesium salt according to the processes cited in U.S. Patent 4,483,781, Hartman (Nov. 20, 1984).

EXAMPLE IV

Equivalent amounts of magnesium hydroxide, hydrogen peroxide (98%), and lauroyl peroxide are added to a reaction vessel containing ethyl acetate and 10 mole % of tetra-n-butylammonium hydroxide and 90 mole % of sodium hydroxide. After 24h of agitation, the reaction mixture is filtered to give a high yield of magnesium bis(peroxylaurate) tetrahydrate and residual phase transfer catalyst. The product may be formulated with laundry detergent ingredients to give an effective granular detergent bleaching composition.

EXAMPLE V

Equivalent amounts of sodium peroxide and magnesium hydroxide are added to a solution of dichloromethane containing an equivalent amount (50 mole %) of benzoic anhydride and 10 mole % of tetra-n-butyl-phosphonium hydroxide. After 48h, cold water is added. After 1h additional, the reaction mixture is filtered to give a high yield of magnesium bis (4-peroxybenzoate) tetrahydrate. This process also is useful in one step preparations of the hydrated magnesium salts of substituted peroxybenzoic acids such as those described in U.S. Patent No. 3,075,921, Brocklehurst and Pengilly (Jan. 29, 1963), incorporated herein by reference. The products, when added to detergent products, remain stable and provide additional stain removal and whitening capability.

EXAMPLE VI

A solution of equivalent amounts of magnesium hydroxide and sodium peroxide in water is added to a solution of an equivalent amount (50 mole %) of 4-t-butylbenzoyl chloride and 20 mole % tetra-n-butylphosphonium bisulfate. After 72h of vigorous stirring, the reaction mixture is cooled and filtered to give a high yield of magnesium bis(4-t-butylperoxybenzoate) tetrahydrate

and minor amounts of the corresponding diacyl peroxide and residual phase transfer catalyst and magnesium salts. The wet filter cake is then intermixed with sodium polyacrylates (MW 2000-10,000 g/mole) and dried to 10% moisture using a fluidized bed to give stable, readily dispersable bleaching granules.

CLAIMS

1. A method for preparation of a peroxycarboxylic acid which comprises reacting a peroxyacid precursor with a source of peroxide in a non-reactive organic solvent in the presence of a phase transfer catalyst.

2. A method according to Claim 1 wherein the phase transfer catalyst is selected from the group consisting of quaternary ammonium and phosphonium salts of Group VA elements, and polyethers.

3. A method according to Claim 2 wherein the phase transfer catalyst is selected from the group consisting of tetra-*n*-butyl ammonium hydroxide, nitrate and bisulfate and tetraethylene glycol dimethyl ether.

4. A method according to Claim 2 wherein the solvent is selected from the group consisting of ethyl acetate, carbon tetrachloride, tetrahydrofuran, benzene, and diethylether.

5. A method according to Claim 2 wherein the peroxyacid precursor is 4-chlorobenzoyl chloride or 4-*t*-butylbenzoyl chloride.

6. A method for preparation of a magnesium peroxycarboxylate which comprises reacting a peroxyacid precursor preferably selected from the group consisting of acyl chlorides and anhydrides with hydrogen or sodium peroxide and a source of magnesium in a nonactive organic solvent in the presence of a phase transfer catalyst.

7. A method according to Claim 6 wherein the phase transfer catalyst is selected from the group consisting of quaternary ammonium and phosphonium salts of Group VA elements, and polyethers.

8. A method according to Claim 7 wherein the phase transfer catalyst is selected from the group consisting of tetra-n-butyl ammonium hydroxide, nitrate and bisulfate, and tetraethylene glycol dimethyl ether.

9. A method according to Claim 8 wherein the solvent is selected from the group consisting of ethyl acetate, carbon tetrachloride, hydrofuran, benzene and diethyl ether.

10. A method according to Claim 9 wherein the peroxyacid precursor is 4-chlorobenzoyl chloride.

11. A method according to Claim 10 wherein the phase transfer catalyst is selected from the group consisting of quaternary ammonium and phosphonium salts of Group VA elements, and polyethers.

DPM:sp(A71/A21)

0206740

European Patent Office

# EUROPEAN SEARCH REPORT

Application number
EP 86304664.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP - A1 - 0 027 693 (INTEROX)<br>* Abstract; examples 1-10 * | 6,7,9, 11 | C 07 C 179/10<br>C 07 C 178/00 |
| D,Y | US - A - 3 992 432 (NAPIER et al.)<br>* Abstract; examples 18-38 * | 6,7,9, 11 | |
| X | DE - B - 1 237 568 (PITTSBURGH PLATE GLASS)<br>* Example 1; column 4, lines 35-37; column 2, line 37 - column 3, line 3 * | 1,2,4 | |
| P,X | EP - A2 - 0 168 204 (CLOROX COMPANY)<br>* Page 7, lines 15-27, especially line 19; claims 2,9 * | 1,2 | **TECHNICAL FIELDS SEARCHED (Int. Cl.4)** |
| A | DE - C - 409 779 (BERGMANN et al.)<br>* Example * | 1 | C 07 C 179/00<br>C 07 C 178/00 |
| A | EP - A2 - 0 105 689 (PROCTER & GAMBLE)<br>* Examples 1,2,4; page 3, lines 10-16 * | 6 | |
| D,A | & US-A-4 483 781 | | |

The present search report has been drawn up for all claims

| Place of search | Date of completion of the search | Examiner |
|---|---|---|
| VIENNA | 18-09-1986 | KÖRBER |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document